# EUROPEAN PATENT SPECIFICATION

(11) **EP 3 601 256 B1**
(45) Date of publication and mention of the grant of the patent: **28.10.2026**
(21) Application number: 18723386.1
(22) Date of filing: 29.03.2018
(51) Int. Cl.: C07D 401/12, C07D 209/54, A61K 31/404, A61P 9/00

(54) **IP6K INHIBITORS**
IP6K-INHIBITOREN
INHIBITEURS D'IP6K

(30) Priority: 30.03.2017 JP 2017066579
(43) Date of publication of application: 05.02.2020
(73) Proprietor: Takeda Pharmaceutical Company Limited, Osaka-shi, Osaka 541-0045 (JP)
(72) Inventor: TERAO, Yoshito, Fujisawa-shi Kanagawa 251-0012 (JP); TAKAHASHI, Masashi, Fujisawa-shi Kanagawa 251-0012 (JP); HARA, Ryoma, Fujisawa-shi Kanagawa 251-0012 (JP); HIDAKA, Kousuke, Fujisawa-shi Kanagawa 251-0012 (JP); FURUKAWA, Hideki, Fujisawa-shi Kanagawa 251-0012 (JP); YAMASAKI, Takeshi, Fujisawa-shi Kanagawa 251-0012 (JP); KASAI, Shizuo, Fujisawa-shi Kanagawa 251-0012 (JP)
(74) Representative: Hoffmann Eitle
(86) International application number: PCT/JP2018/014502
(87) International publication number: WO 2018/182051

(56) References cited:
- EP-A1- 0 178 876
- WO-A2-2004/028459
- YUSUKE MORITOH ET AL: "Inositol Hexakisphosphate Kinase 3 Regulates Metabolism and Lifespan in Mice", SCIENTIFIC REPORTS, vol. 6, no. 1, 31 August 2016 (2016-08-31), XP055490028, DOI: 10.1038/srep32072

## Description

### [Technical Field]

The present invention relates to a heterocyclic compound having an inositol hexakisphosphate kinase (Inositol hexakisphosphate kinase) (sometimes to be abbreviated as "IP6K" in the present specification) inhibitory action and expected to be useful as a prophylactic or therapeutic agent for diseases such as cardiac failure (patent document 1, diabetes and the like.

### (Background of the Invention)

IP6K is a molecule involved in many intracellular signaling by generating inositol pyrophosphate (non-patent documents 1, 2). Inositol pyrophosphate is a low molecule with high energy and the production thereof is known to be mediated by two kinds of enzymes. The two kinds are IP6K having 5 kinase activity (non-patent documents 3-5) and diphosphoinositol pentakisphosphate kinase having 1 kinase activity (PPIP5K (non-patent documents 6-8)).

The 5 kinase activity of IP6K produces 5-PP-InsP₄ from myo-inositol (1,3,4,5,6)-pentakisphosphate (InsP₅), 5-PP-InsP₅ from InsP₆ (5-InsP₇ (non-patent document 9), hereinafter to be indicated as IP7), and 1,5-PP2-InsP₄ (InsP₈) from 1-PP-InsP₅. Among these, IP7 is the most studied inositol pyrophosphate produced by IP6K and controls many biological reactions by directly binding to the target protein or through the mechanism of pyrophosphorylation (transfer of β-phosphoric acid of pyrophosphoric acid moiety of IP7 to phosphorylated serine residue of target protein) (non-patent document 10). IP7 produced by 5PPIP5K is 1-InsP₇, almost all parts of IP7 in mammals are considered to be 5-isomer 5-InsP₇ (non-patent documents 3, 11).

Mammals have three IP6K subtypes of IP6K1, IP6K2, and IP6K3 (non-patent document 1), and recent studies using knockout mice have suggested the physiological role of each molecule and possibility as the target of drug discovery. The knockout mouse of IP6K1 has increased Akt signal in the liver, fat and muscle, and improvement of glucose tolerance, promotion of insulin sensitivity and increase in muscle amount have been confirmed under high-fat diet loading conditions (non-patent document 12). On the other hand, IP7 produced by IP6K2 has been reported to function as a major mediating molecule in cancer cell migration and tumor metastasis, and it has been confirmed that cellular infiltration and metastasis are suppressed by deficiency of IP6K2 in cancer cells (non-patent document 13). In addition, IP6K2 has been confirmed to have an action to promote cell death by producing IP7, and relation to neurodegenerative diseases such as amyotrophic lateral sclerosis and the like has been pointed out (non-patent document 14). IP6K3 is a molecule highly expressed in muscles. It has been confirmed that the gene expression thereof is induced by fasting and diabetic condition, the knockout mouse shows low blood glucose concentration and low blood insulin concentration, shows promoted glucose tolerance and promoted insulin sensitivity, and has a long life (non-patent document 15).

### [Document List]

### [non-patent document]

non-patent document 1: Cell Mol Life Sci 66, 3851-3871,
   doi:10.1007/s00018-009-0115-2 (2009).
non-patent document 2: Adv Enzyme Regul 51, 74-82,
   doi:10.1016/j.advenzreg.2010.08.003 (2011).
non-patent document 3: J Biol Chem 284, 1863-1872,
   doi:10.1074/jbc.M805686200 (2009).
non-patent document 4: Curr Biol 9, 1323-1326 (1999).
non-patent document 5: Chem Biol 15, 274-286,
   doi:10.1016/j.chembiol.2008.01.011 (2008).
non-patent document 6: J Biol Chem 282, 30763-30775,
   doi:10.1074/jbc.M704655200 (2007).
non-patent document 7: J Biol Chem 282, 30754-30762,
   doi:10.1074/jbc.M704656200 (2007).
non-patent document 8: Nat Chem Biol 8, 111-116, doi:10.1038/n chembio.733 (2012).
non-patent document 9: Adv Biol Regul 57, 203-216,
   doi:10.1016/j.jbior.2014.09.015 (2015).
non-patent document 10: Biochem J 452, 369-379,
   doi:10.1042/BJ20130118 (2013).
non-patent document 11: Biochem J 327 (Pt 2), 553-560 (1997).
non-patent document 12: Cell 143, 897-910,
   doi:10.1016/j.cell.2010.11.032 (2010).
non-patent document 13: Proc Natl Acad Sci U S A 112, 1773-1778,
   doi:10.1073/pnas.1424642112 (2015).
non-patent document 14: Mol Neurobiol 53, 5377-5383,
   doi:10.1007/s12035-015-9470-1 (2016).
non-patent document 15: Sci Rep 6, 32072, doi:10.1038/srep32072

(2016). Patent document 1: EP 0 178 876 A1

### [SUMMARY OF THE INVENTION]

### [Problems to be Solved by the Invention]

The present invention aims to provide a heterocyclic compound having an IP6K inhibitory action and expected to be useful as a prophylactic or therapeutic agent for diseases such as cardiac failure, diabetes and the like.

### [Means of Solving the Problems]

The present inventors have found that the present invention have identified a compound having a specific structure, in particular 4-((3,5-dichloropyridin-2-yl)oxy)-2'-oxo-1',2'-dihydrospiro[cyclohexane-1,3'-indole]-5'-carboxylic acid, or a salt thereof (sometimes to be referred to asthe compound of the present invention or compound (I) in the present specification) has an IP6K inhibitory action, and conducted further studies, which resulted in the completion of the present invention.

Accordingly, the present invention is as described in the claims.

### [Effect of the Invention]

The compound of the present invention has an IP6K inhibitory action, and is expected to be useful as a prophylactic or therapeutic agent for diseases such as cardiac failure, diabetes and the like.

### [Brief Description of the Drawings]

Fig. 1 shows a molecular structure of the compound of Example 1, obtained by single crystal X-ray structure analysis.
Fig. 2 shows IP7 levels of the liver, muscle and fat of a rat by administration of compound 97.
Fig. 3 shows plasma glucose of a diabetes model rat for up to 24 hr after oral administration.
Fig. 4 shows glucose AUC (area under curve) of a diabetes model rat for up to 24 hr after oral administration.
Fig. 5 shows blood glucose of a rat with impaired glucose tolerance for up to 120 min after administration of compound 9.
Fig. 6 shows blood glucose during fasting of a rat with impaired glucose tolerance after administration of compound 9.
Fig. 7 shows glucose AUC of a rat with impaired glucose tolerance for up to 120 min after administration of compound 9.
Fig. 8 shows correlation between glucose AUC and IP7 level of muscle of a rat with impaired glucose tolerance after administration of compound 9.
Fig. 9 shows correlation between glucose AUC and IP7 level of the liver of a rat with impaired glucose tolerance after administration of compound 9.
Fig. 10 shows the survival rate of male CSQ TG mouse after administration of compound 97 and compound 9.

### (Detailed Description of the Invention)

When compound (I) is in a form of a salt, examples thereof include metal salts, an ammonium salt, salts with organic base, salts with inorganic acid, salts with organic acid, salts with basic or acidic amino acid, and the like. Preferable examples of the metal salt include alkali metal salts such as sodium salt, potassium salt and the like; alkaline earth metal salts such as calcium salt, magnesium salt, barium salt and the like; an aluminum salt, and the like. Preferable examples of the salt with organic base include salts with trimethylamine, triethylamine, pyridine, picoline, 2,6-lutidine, ethanolamine, diethanolamine, triethanolamine, cyclohexylamine, dicyclohexylamine, N,N'-dibenzylethylenediamine and the like. Preferable examples of the salt with inorganic acid include salts with hydrochloric acid, hydrobromic acid, nitric acid, sulfuric acid, phosphoric acid and the like. Preferable examples of the salt with organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid and the like. Preferable examples of the salt with basic amino acid include salts with arginine, lysine, ornithine and the like. Preferable examples of the salt with acidic amino acid include salts with aspartic acid, glutamic acid and the like.

Among them, a pharmaceutically acceptable salt is preferable. For example, when a compound has an acidic functional group, examples thereof include inorganic salts such as alkali metal salts (e.g., sodium salt, potassium salt etc.), alkaline earth metal salts (e.g., calcium salt, magnesium salt etc.) and the like, ammonium salt etc., and when a compound has a basic functional group, examples thereof include salts with inorganic acid such as hydrochloric acid, hydrobromic acid, nitric acid, sulfuric acid, phosphoric acid and the like, and salts with organic acid such as acetic acid, phthalic acid, fumaric acid, oxalic acid, tartaric acid, maleic acid, citric acid, succinic acid, methanesulfonic acid, benzenesulfonic acid, p-toluenesulfonic acid and the like.

The production method of the compound of the present invention is explained in the followings.

The raw material compound and reagent used and the compound obtained in each step in the following production method may be each in a form of a salt, and examples of such salt include those similar to the salts of the compound of the present invention and the like.

When the compound obtained in each step is a free form, it can be converted to the objective salt according to a method known *per se.* When the compound obtained in each step is a salt, it can be converted to the free form or the objective other salt according to a method known *per se.*

The compound obtained in each step can be used directly as the reaction solution or as a crude product for the next reaction. Alternatively, the compound obtained in each step can be isolated and purified from a reaction mixture according to a method known *per se,* for example, a separation means such as concentration, crystallization, recrystallization, distillation, solvent extraction, fractional distillation, column chromatography and the like.

When the raw material compound and reagent used in each step are commercially available, the commercially available product can also be used directly.

In the reaction in each step, while the reaction time varies depending on the kind of the reagent and solvent to be used, it is generally 1 min - 48 hr, preferably 10 min - 8 hr, unless otherwise specified.

In the reaction in each step, while the reaction temperature varies depending on the kind of the reagent and solvent to be used, it is generally -78°C - 300°C, preferably - 78°C - 150°C, unless otherwise specified.

In the reaction in each step, while the pressure varies depending on the kind of the reagent and solvent to be used, it is generally 1 atm - 20 atm, preferably 1 atm - 3 atm, unless otherwise specified.

Microwave synthesizer such as Initiator manufactured by Biotage and the like may be used for the reaction in each step. While the reaction temperature varies depending on the kind of the reagent and solvent to be used, it is generally room temperature - 300°C, preferably 50°C - 250°C, unless otherwise specified. While the reaction time varies depending on the kind of the reagent and solvent to be used, it is generally 1 min - 48 hr, preferably 1 min - 8 hr, unless otherwise specified.

In the reaction in each step, the reagent is used in an amount of 0.5 equivalents - 20 equivalents, preferably 0.8 equivalents - 5 equivalents, relative to the substrate, unless otherwise specified. When the reagent is used as a catalyst, the reagent is used in an amount of 0.001 equivalent - 1 equivalent, preferably 0.01 equivalent - 0.2 equivalent, relative to the substrate. When the reagent is used as a reaction solvent, the reagent is used in a solvent amount.

Unless otherwise specified, the reaction in each step is carried out without solvent, or by dissolving or suspending the raw material compound in a suitable solvent. Examples of the solvent include those described in Examples and the following solvents.
alcohols: methanol, ethanol, tert-butyl alcohol, 2-methoxyethanol and the like;
ethers: diethyl ether, diphenyl ether, tetrahydrofuran, 1,2-dimethoxyethane and the like;
aromatic hydrocarbons: chlorobenzene, toluene, xylene and the like;
saturated hydrocarbons: cyclohexane, hexane and the like; amides: N,N-dimethylformamide, N-methylpyrrolidone and the like;
halogenated hydrocarbons: dichloromethane, carbon tetrachloride and the like;
nitriles: acetonitrile and the like;
sulfoxides: dimethyl sulfoxide and the like;
aromatic organic bases: pyridine and the like;
anhydrides: acetic anhydride and the like;
organic acids: formic acid, acetic acid, trifluoroacetic acid and the like;
inorganic acids: hydrochloric acid, sulfuric acid and the like; esters: ethyl acetate and the like;
ketones: acetone, methyl ethyl ketone and the like;
water.

The above-mentioned solvent can be used in a mixture of two or more kinds thereof in an appropriate ratio.

When a base is used for the reaction in each step, examples thereof include those described in Examples and the following bases.
inorganic bases: sodium hydroxide, magnesium hydroxide, sodium carbonate, calcium carbonate, sodium hydrogen carbonate and the like;
organic bases: triethylamine, diethylamine, pyridine, 4-dimethylaminopyridine, N,N-dimethylaniline, 1,4-diazabicyclo[2.2.2]octane, 1,8-diazabicyclo[5.4.0]-7-undecene, imidazole, piperidine and the like;
metal alkoxides: sodium ethoxide, potassium tert-butoxide and the like;
alkali metal hydrides: sodium hydride and the like;
metal amides: sodium amide, lithium diisopropylamide, lithium hexamethyldisilazide and the like;
organic lithiums: n-butyllithium and the like.

When an acid or an acid catalyst is used for the reaction in each step, examples thereof include those described in Examples and the following acids and acid catalysts.
inorganic acids: hydrochloric acid, sulfuric acid, nitric acid, hydrobromic acid, phosphoric acid and the like;
organic acids: acetic acid, trifluoroacetic acid, citric acid, p-toluenesulfonic acid, 10-camphorsulfonic acid and the like; Lewis acid: boron trifluoride diethyl ether complex, zinc iodide, anhydrous aluminum chloride, anhydrous zinc chloride, anhydrous iron chloride and the like.

Unless otherwise specified, the reaction in each step is carried out according to a method known *per se,* for example, the method described in Jikken Kagaku Kouza, 5th Edition, vol.13-19 (the Chemical Society of Japan ed.); Shin Jikken Kagaku Kouza, vol.14-15 (the Chemical Society of Japan ed.); Fine Organic Chemistry, Revised 2nd Edition (L. F. Tietze, Th. Eicher, Nankodo); Organic Name Reactions, the Reaction Mechanism and Essence, Revised Edition (Hideo Togo, Kodansha); ORGANIC SYNTHESES Collective Volume I-VII (John Wiley & SonsInc); Modern Organic Synthesis in the Laboratory A Collection of Standard Experimental Procedures (Jie Jack Li, OXFORD UNIVERSITY); Comprehensive Heterocyclic Chemistry III, Vol.1 -Vol.14 (Elsevier Japan); Strategic Applications of Named Reactions in Organic Synthesis (translated by Kiyoshi Tomioka, Kagakudojin); Comprehensive Organic Transformations (VCH Publishers Inc.), 1989, or the like, or the method described in Examples.

In each step, the protection or deprotection reaction of an functional group is carried out according to a method known *per se,* for example, the method described in "Protective Groups in Organic Synthesis, 4th Ed", Wiley-Interscience, Inc., 2007 (Theodora W. Greene, Peter G. M. Wuts); "Protecting Groups 3rd Ed." Thieme, 2004 (P.J. Kocienski), or the like, or the method described in Examples.

Examples of the protecting group for hydroxyl group such as alcohol or phenolic hydroxyl group include ether type protecting groups such as methoxymethyl ether, benzylether, tert-butyldimethylsilylether, tetrahydropyranylether and the like; carboxylate type protecting groups such as acetic acid ester and the like; sulfonic acid ester type protecting groups such as methanesulfonic acid ester and the like; carbonate type protecting groups such as tert-butylcarbonate and the like, and the like.

Examples of the protecting group for carbonyl group of aldehyde include acetal type protecting groups such as dimethylacetal and the like; cyclic acetal type protecting groups such as 1,3-dioxane and the like, and the like.

Examples of the protecting group for carbonyl group of ketone include ketal-type protecting groups such as dimethyl ketal and the like; cyclic ketal-type protecting groups such as 1,3-dioxane and the like; oxime-type protecting groups such as O-methyloxime and the like; hydrazone-type protecting groups such as N,N-dimethylhydrazone and the like, and the like.

Examples of the protecting group for carboxyl group include ester-type protecting groups such as methyl ester and the like; amide-type protecting groups such as N,N-dimethylamide and the like, and the like.

Examples of the protecting group for thiol include ether-type protecting groups such as benzylthioether and the like; ester-type protecting groups such as thioacetic acid ester, thiocarbonate, thiocarbamate and the like, and the like.

Examples of the protecting group for amino group and aromatic hetero ring such as imidazole, pyrrole, indole and the like include carbamate-type protecting groups such as benzylcarbamate and the like; amide-type protecting groups such as acetamide and the like; alkylamine-type protecting groups such as N-triphenylmethylamine and the like, sulfonamide-type protecting groups such as methanesulfonamide and the like, and the like.

The protecting groups can be removed according to a method known *per se,* for example, by employing a method using acid, base, ultraviolet rays, hydrazine, phenylhydrazine, sodium N-methyldithiocarbamate, tetrabutylammonium fluoride, palladium acetate, trialkylsilyl halide (e.g., trimethylsilyl iodide, trimethylsilyl bromide) and the like, a reduction method, and the like.

The compound of the present invention can be produced by the method described in Examples. The compound of the present invention may have an optical isomer a stereoisomer, a regioisomer of a totamer, and these are also encompassed in the compound of the present invention. It can be obtained as a single product according to a synthesis method and separation method known *per se* (e.g., concentration, solvent extraction, column chromatography, recrystallization etc.). For example, when compound (I) has an optical isomer, the optical isomer resolved from the compound is also encompassed in compound (I).

The optical isomer can be produced according to a method known *per se.* Specifically, the optical isomer is obtained using an optically active synthetic intermediate or by subjecting the racemic final product to an optical resolution according to a known method.

The optical resolution may be a method known *per se,* such as a fractional recrystallization method, a chiral column method, a diastereomer method etc.

### 1) Fractional recrystallized method

A method wherein a salt of a racemate with an optically active compound (e.g., (+)-mandelic acid, (-)-mandelic acid, (+)-tartaric acid, (-)-tartaric acid, (+)-1-phenethylamine, (-)-1-phenethylamine, cinchonine, (-)-cinchonidine, brucine etc.) is formed, which is separated by a fractional recrystallized method, and if desired, a neutralization step to give a free optical isomer.

### 2) Chiral column method

A method wherein a racemate or a salt thereof is applied to a column (a chiral column) for separation of an optical isomer to allow separation. In the case of a liquid chromatography, for example, a mixture of the optical isomers is applied to a chiral column such as ENANTIO-OVM (manufactured by Tosoh Corporation), CHIRAL series (manufactured by Daicel Chemical Industries, Ltd.) and the like, and developed with water, various buffers (e.g., phosphate buffer, etc.) and organic solvents (e.g., ethanol, methanol, 2-propanol, acetonitrile, trifluoroacetic acid, diethylamine, etc.) as an eluent, solely or in admixture to separate the optical isomer. In the case of a gas chromatography, for example, a chiral column such as CP-Chirasil-DeX CB (manufactured by GL Sciences Inc.) and the like is used to allow separation.

### 3) Diastereomer method

A method wherein a racemic mixture is prepared into a diastereomeric mixture by chemical reaction with an optically active reagent, which is made into a single substance by a typical separation means (e.g., a fractional recrystallized method, a chromatography method etc.) and the like, and is subjected to a chemical treatment such as hydrolysis and the like to separate an optically active reagent moiety, whereby an optical isomer is obtained. When compound has a carboxy group, the compound and an optically active amine or an optically active alcohol reagent are subjected to condensation reaction to give diastereomers of the amide compound or the ester compound, respectively. The separated diastereomer is converted to an optical isomer of the original compound by acid hydrolysis or base hydrolysis.

Compound (I) may be a crystal.

The crystal of compound (I) can be produced according to a crystallization method known *per se.*

Examples of the crystallization method include crystallization method from a solution, crystallization method from vapor, crystallization method from a melt, and the like.

The "crystallization method from a solution" is typically a method of shifting a non-saturated state to supersaturated state by varying factors involved in solubility of compounds (solvent composition, pH, temperature, ionic strength, redox state, etc.) or the amount of solvent. Specific examples thereof include a concentration method, a slow cooling method, a reaction method (a diffusion method, an electrolysis method), a hydrothermal growth method, a flux method and the like. Examples of the solvent to be used include aromatic hydrocarbons (e.g., benzene, toluene, xylene, etc.), halogenated hydrocarbons (e.g., dichloromethane, chloroform, etc.), saturated hydrocarbons (e.g., hexane, heptane, cyclohexane, etc.), ethers (e.g., diethyl ether, diisopropyl ether, tetrahydrofuran, 1,4-dioxane, etc.), nitriles (e.g., acetonitrile, etc.), ketones (e.g., acetone, etc.), sulfoxides (e.g., dimethyl sulfoxide, etc.), acid amides (e.g., N,N-dimethylformamide, etc.), esters (e.g., ethyl acetate, etc.), alcohols (e.g., methanol, ethanol, 2-propanol, etc.), water and the like. These solvents are used alone or in a combination of two or more at a suitable ratio (e.g., 1:1 to 1:100 (a volume ratio)). Where necessary, a seed crystal can be used.

The "crystallization method from vapor" is, for example, a vaporization method (a sealed tube method, a gas stream method), a gas phase reaction method, a chemical transportation method and the like.

The "crystallization method from a melt" is, for example, a normal freezing method (a pulling method, a temperature gradient method, a Bridgman method), a zone melting method (a zone leveling method, a floating zone method), a special growth method (a VLS method, a liquid phase epitaxy method) and the like.

Preferable examples of the crystallization method include a method comprising dissolving compound (I) in a suitable solvent (e.g., alcohols such as methanol, ethanol etc.) at 20°C to 120°C, and cooling the obtained solution to a temperature (e.g., 0 to 50°C, preferably 0 to 20°C) not higher than the dissolution temperature, and the like.

The thus-obtained crystals of the present invention can be isolated, for example, by filtration and the like.

An analysis method of the obtained crystal is generally a method of crystal analysis by powder X-ray diffraction. As a method of determining crystal orientation, a mechanical method or an optical method and the like can also be used.

The crystal of compound (I) obtained by the above-mentioned production method may have high purity, high quality, and low hygroscopicity, may not be denatured even after a long-term preservation under general conditions, and may be expected to be superior in the stability. In addition, it may be also superior in the biological properties (e.g., pharmacokinetics (absorption, distribution, metabolism, excretion), efficacy expression etc.) and may be extremely useful as a medicament.

The prodrug of compound (I) means a compound which is converted to compound (I) with a reaction due to an enzyme, gastric acid and the like under the physiological condition in the living body, that is, a compound which is converted to compound (I) by enzymatic oxidation, reduction, hydrolysis and the like; a compound which is converted to compound (I) by hydrolysis and the like due to gastric acid, and the like. Examples of the prodrug for compound (I) include a compound obtained by subjecting an amino group in compound (I) to acylation, alkylation or phosphorylation (e.g., a compound obtained by subjecting an amino group in compound (I) to eicosanoylation, alanylation, pentylaminocarbonylation, (5-methyl-2-oxo-1,3-dioxolen-4-yl)methoxycarbonylation, tetrahydrofurylation, pyrrolidylmethylation, pivaloyloxymethylation, tert-butylation, and the like); a compound obtained by subjecting a hydroxy group in compound (I) to acylation, alkylation, phosphorylation or boration (e.g., a compound obtained by subjecting a hydroxy group in compound (I) to acetylation, palmitoylation, propanoylation, pivaloylation, succinylation, fumarylation, alanylation or dimethylaminomethylcarbonylation, and the like); a compound obtained by subjecting a carboxyl group in compound (I) to esterification or amidation (e.g., a compound obtained by subjecting a carboxyl group in compound (I) to ethyl esterification, phenyl esterification, carboxymethyl esterification, dimethylaminomethyl esterification, pivaloyloxymethyl esterification, ethoxycarbonyloxyethyl esterification, phthalidyl esterification, (5-methyl-2-oxo-1,3-dioxolen-4-yl)methyl esterification, cyclohexyloxycarbonylethyl esterification or methylamidation, and the like) and the like. These compounds can be produced from compound (I) according to a method known *per se.*

The prodrug of compound (I) may also be one which is converted to compound (I) under physiological conditions as described in "IYAKUHIN no KAIHATSU (Development of Pharmaceuticals)", Vol. 7, Design of Molecules, p. 163-198, Published by HIROKAWA SHOTEN (1990).

Compound (I) may be a hydrate, a non-hydrate, a solvate or a non-solvate.

In addition, compound (I) may be a compound labeled or substituted with an isotope (e.g., ²H, ³H, ¹¹C, ¹⁴C, ¹⁸F, ³⁵S, ¹²⁵I and the like) and the like. The compound labeled or substituted with an isotope may be used, for example, as a tracer (PET tracer) used in positron emission tomography (PET), and useful in the field of medical diagnosis and the like.

Compound (I) also encompasses a deuterium conversion form wherein ¹H is converted to ²H(D).

Compound (I) also encompasses a tautomer thereof.

Compound (I) may be a pharmaceutically acceptable cocrystal or a salt thereof. The cocrystal or a salt thereof means a crystalline substance constituted with two or more special solids at room temperature, each having different physical properties (e.g., structure, melting point, melting heat, hygroscopicity, solubility and stability etc.). The cocrystal or a salt thereof can be produced according to a cocrystallization a method known *per se.*

Compound (I) may also be used as a PET tracer.

Since the compound of the present invention has a superior IP6K inhibitory action, it may also be useful as a safe medicament based on this action.

For example, the medicament of the present invention containing the compound of the present invention may be expected to show low toxicity (e.g., acute toxicity, chronic toxicity, genetic toxicity, reproductive toxicity, cardiotoxicity, carcinogenicity), and used for a mammal (e.g., mouse, rat, hamster, rabbit, cat, dog, bovine, sheep, monkey, human etc.) as a prophylactic or therapeutic agent for diseases involving IP6K.

To be specific, the compound of the present invention can be used as an agent for the prophylaxis or treatment of diabetes (e.g., type 1 diabetes, type 2 diabetes, gestational diabetes, obese diabetes), cardiovascular disease (e.g., cardiac failure, arrhythmia, ischemic cardiac diseases, heart valvular disease, arteriosclerosis), obesity (e.g., malignant mastocytosis, exogenous obesity, hyperinsulinar obesity, hyperplasmic obesity, hypophyseal adiposity, hypoplasmic obesity, hypothyroid obesity, hypothalamic obesity, symptomatic obesity, infantile obesity, upper body obesity, alimentary obesity, hypogonadal obesity, systemic mastocytosis, simple obesity, central obesity etc.), non-alcoholic fatty liver diseases (NAFLD), non-alcoholic steatohepatitis (NASH), hyperphagia, hyperlipidemia/dyslipidemia (e.g., hypertriglyceridemia, hypercholesterolemia, high LDL-cholesterolemia, hypo HDL-cholesterolemia, postprandial hyperlipemia), Hyperphosphatemia, hypophosphatemia, Hyperkalemia, hypertension, diabetic complications [e.g., neuropathy, nephropathy, retinopathy, diabetic cardiomyopathy, cataract, macroangiopathy, osteopenia, hyperosmolar diabetic coma, infections (e.g., respiratory infection, urinary tract infection, gastrointestinal infection, dermal soft tissue infections, inferior limb infection), diabetic gangrene, xerostomia hypacusis, hypacusis, cerebrovascular diseases, peripheral blood circulation disorder], metabolic syndrome (pathology with not less than 3 selected from hyper-triglycerid(TG)emia, hypo HDL cholesterol(HDL-C)emia, hypertension, abdomen obesity and impaired glucose tolerance), sarcopenia, emotional disorder, sexual dysfunction, depression, anxiety, neurosis, arteriosclerosis, gonitis, acute renopathy, glaucoma, ischemic disease, myocardial infarction, cerebral apoplexy, dementia, Neurodegenerative diseases [e.g., amyotrophic lateral sclerosis], mitochondria disease, Retinitis pigmentosa, glaucoma, Osteoporosis, fungi infections and the like.

For diagnostic criteria of diabetes, Japan Diabetes Society issued "Report of the Committee on the Classification and Diagnostic Criteria of Diabetes Mellitus" in 2010.

According to this report, diabetes is a condition showing any of a fasting blood glucose level (glucose concentration of intravenous plasma) of not less than 126 mg/dL, a 75 g oral glucose tolerance test (75 g OGTT) 2 h level (glucose concentration of intravenous plasma) of not less than 200 mg/dL, a non-fasting blood glucose level (glucose concentration of intravenous plasma) of not less than 200 mg/dL and HbA1c (international standard value) of not less than 6.5%. HbA1c (international standard value) (%) is indicated as a value equivalent to the internationally standardized NGSP (National Glycohemoglobin Standardization Program), which is a value of HbA1c (JDS value) (%) expressed by conventional JDS (Japan Diabetes Society) value plus 0.4% thereof. A condition not falling under the above-mentioned diabetes and different from "a condition showing a fasting blood glucose level (glucose concentration of intravenous plasma) of less than 110 mg/dL or a 75 g oral glucose tolerance test (75 g OGTT) 2 h level (glucose concentration of intravenous plasma) of less than 140 mg/dL" (normal type) is called a "borderline type".

According to the reports from WHO (World Health Organization) in 2006, diabetes is a condition showing a fasting blood glucose level (glucose concentration of intravenous plasma) of not less than 126 mg/dL, or a 75 g oral glucose tolerance test 2 h level (glucose concentration of intravenous plasma) of not less than 200 mg/dL.

According to the above-mentioned reports, impaired glucose tolerance (IGT) is a condition showing a fasting blood glucose level (glucose concentration of intravenous plasma) of not less than 126 mg/dL and a 75 g oral glucose tolerance test 2 h level (glucose concentration of intravenous plasma) of not less than 140 mg/dL and less than 200 mg/dL. According to the report of WHO, a condition showing a fasting blood glucose level (glucose concentration of intravenous plasma) of not less than 110 mg/dL and less than 126 mg/dL and, if a measurement value exists, a 75 g oral glucose tolerance test 2 h level (glucose concentration of intravenous plasma) of less than 140 mg/dL is called IFG (Impaired Fasting Glucose).

The compound of the present invention can also be used as an agent for the prophylaxis or treatment of diabetes, borderline type, impaired glucose tolerance or IFG (Impaired Fasting Glucose), as judged according to the above-mentioned reports. Moreover, the compound of the present invention can also prevent progress of borderline type, impaired glucose tolerance or IFG (Impaired Fasting Glucose) into diabetes.

The clinical condition of cardiac failure is classified into 4 stages shown in Table 1 according to the severity by the New York Heart Association (NYHA).

**Table 1**

| NYHA classification of cardiac failure | |
|---|---|
| level | symptom of patient |
| level I | Cardiac disease, but no limitation of physical activity. Ordinary physical activity does not cause undue fatigue, palpitation, dyspnea or anginal pain. |
| level II | Slight limitation of physical activity. No symptom at rest. Ordinary physical activity results in fatigue, palpitation, dyspnea or angina pain. |
| level III | Marked limitation of physical activity. No symptom at rest. Less than ordinary activity causes fatigue, palpitation, dyspnea or angina pain. |
| level IV | All physical activities are limited due to cardiac disease. |
| | Cardiac failure symptoms and angina pain exist even at rest. A small effort aggravates these symptoms. |

| | |
|---|---|
| level IIs: Slight limitation of physical activity level IIm: Moderate limitation of physical activity | |

The AHA/ACC stage classification (American Heart Association/American college of cardiology) divides into 4 stages shown in Table 2 according to the severity.

**Table 2**

| AHA/ACC stage classification of cardiac failure | |
|---|---|
| stage | definition |
| A | with risk factor but no cardiac dysfunction |
| B | left ventricular systolic dysfunction without symptom |
| C | symptomatic heart failure |
| D | intractable cardiac failure |

The NYHA classification and AHA/ACC stage classification generally correspond to each other as shown in the following Table 3.

**Table 3**

| corresponding relation between NYHA classification and AHA/ACC stage classification | |
|---|---|
| NYHA classification | AHA/ACC stage classification |
| - | A |
| level I | B |
| level II | C |
| level III | |
| level IV | |
| level IV | D |

The compound of the present invention is also used as a prophylactic or therapeutic agent for cardiac failure, low cardiac output, ischemic cardiac failure, non-ischemic cardiac failure, decompensated cardiac failure, acute cardiac failure or acute decompensated cardiac failure, judged by the above-mentioned reports.

The compound of the present invention can also be used for improving low ejection fraction or increasing ejection fraction in subjects with cardiac failure.

The compound of the present invention can also suppress aggravation of cardiac function or suppress progression of aggravation in subjects with cardiac failure. The compound of the present invention can also reduce load on the heart, suppress cardiac hypertrophy, suppress stromafibrosis and suppress increase of apoptosis in subjects with cardiac failure.

The compound of the present invention is also useful as an agent for the prophylaxis or treatment of metabolic syndrome. Because patients with metabolic syndrome have an extreme high incidence of cardiovascular diseases as compared to patients with single lifestyle-related disease, the prophylaxis or treatment of metabolic syndrome is quite important to prevent cardiovascular diseases.

Criteria for diagnosis of metabolic syndrome are announced by WHO in 1999, and by NCEP in 2001. According to the criterion of WHO, patients with at least two of abdominal obesity, dyslipidemia (high TG or low HDL) and hypertension in addition to hyperinsulinemia or impaired glucose tolerance are diagnosed as metabolic syndrome (World Health Organization: Definition, Diagnosis and Classification of Diabetes Mellitus and Its Complications. Part I: Diagnosis and Classification of Diabetes Mellitus, World Health Organization, Geneva, 1999). According to the criterion of Adult Treatment Panel III of National Cholesterol Education Program, that is an indicator for managing ischemic heart diseases in America, patients with at least three of abdominal obesity, hypertriglyceridemia, hypo HDL cholesterolemia, hypertension and impaired glucose tolerance are diagnosed as metabolic syndrome (National Cholesterol Education Program: Executive Summary of the Third Report of National Cholesterol Education Program (NCEP) Expert Panel on Detection, Evaluation, and Treatment of High Blood Cholesterol in Adults (Adults Treatment Panel III). The Journal of the American Medical Association, Vol. 285, 2486-2497, 2001).

The compound of the present invention can also be used for the secondary prevention and the suppression of progression of the above-mentioned various diseases (e.g., cardiovascular event such as myocardial infarction and the like).

Here, the above-mentioned "prophylaxis" of a disease means, for example, administration of a medicament containing the compound of the present invention to patients who are expected to have a high risk of the onset due to some factor relating to the disease but have not developed the disease or patients who have developed the disease but do not have a subjective symptom, or administration of a medicament containing the compound of the present invention to patients who are feared to show recurrence of the disease after treatment of the disease.

A medicament containing the compound of the present invention may be able to use the compound of the present invention solely or as a pharmaceutical composition of the compound of the present invention mixed with a pharmacologically acceptable carrier according to a method known *per se* (e.g., the method described in the Japanese Pharmacopoeia etc.) as the production method of a pharmaceutical preparation. The medicament containing the compound of the present invention may be able to be administered safely as, for example, tablet (including sugar-coated tablet, film-coated tablet, sublingual tablet, orally disintegrating tablet, buccal etc.), pill, powder, granule, capsule (including soft capsule, microcapsule), troche, syrup, liquid, emulsion, suspension, release control preparation (e.g., immediate-release preparation, sustained-release preparation, sustained-release microcapsule), aerosol, film (e.g., orally disintegrating film, oral mucosa-adhesive film), injection (e.g., subcutaneous injection, intravenous injection, intramuscular injection, intraperitoneal injection), drip infusion, transdermal absorption type preparation, cream, ointment, lotion, adhesive preparation, suppository (e.g., rectal suppository, vaginal suppository), pellet, nasal preparation, pulmonary preparation (inhalant), eye drop and the like, orally or parenterally (e.g., intravenous, intramuscular, subcutaneous, intraorgan, intranasal, intradermal, instillation, intracerebral, intrarectal, intravaginal, intraperitoneal and intratumor administrations, administration to the vicinity of tumor and direct administration to the lesion).

The content of the compound of the present invention in the medicament of the present invention is about 0.01 to 100% by weight of the entire medicament. The dose may vary depending on administration subject, administration route, disease and the like. For example, for oral administration to patients (body weight about 60 kg) with diabetes, about 0.01 mg/kg body weight - about 500 mg/kg body weight, preferably about 0.1 mg/kg body weight - about 50 mg/kg body weight, more preferably about 1 mg/kg body weight - about 30 mg/kg body weight of an active ingredient (compound (I)) may be administered once to several portions per day.

The pharmaceutically acceptable carrier, which may be used for the production of the medicament of the present invention, may be exemplified by various organic or inorganic carrier materials that are conventionally used as preparation materials, for example, excipient, lubricant, binding agent and disintegrant for solid preparations; or solvent, solubilizing agent, suspending agent, isotonic agent, buffering agent, soothing agent and the like for liquid preparations. Furthermore, when necessary, ordinary additives such as preservative, antioxidant, colorant, sweetening agent, adsorbing agent, wetting agent and the like can also be used as appropriate in an appropriate amount.

The dose of the pharmaceutical composition of the present invention as a sustained-release preparation varies depending on the kind and content of compound (I), dosage form, period of sustained drug release, subject animal of administration (e.g., mammals such as mouse, rat, hamster, guinea pig, rabbit, cat, dog, bovine, horse, swine, sheep, monkey, human etc.), and administration object. For example, for application by parenteral administration, about 0.1 to about 100 mg of compound (I) needs to be released from the administered preparation per 1 week.

Examples of the excipient include lactose, white sugar, D-mannitol, starch, corn starch, crystalline cellulose, light anhydrous silicic acid and the like.

Examples of the lubricant include magnesium stearate, calcium stearate, talc, colloidal silica and the like.

Examples of the binding agent include crystalline cellulose, white sugar, D-mannitol, dextrin, hydroxypropylcellulose, hydroxypropylmethylcellulose, polyvinylpyrrolidone, starch, sucrose, gelatin, methylcellulose, carboxymethylcellulose sodium and the like.

Examples of the disintegrant include starch, carboxymethylcellulose, carboxymethylcellulose calcium, carboxymethylstarch sodium, L-hydroxypropylcellulose and the like.

Examples of the solvent include water for injection, alcohol, propylene glycol, Macrogol, sesame oil, corn oil, olive oil and the like.

Examples of the solubilizing agent include polyethylene glycol, propylene glycol, D-mannitol, benzyl benzoate, ethanol, trisaminomethane, cholesterol, triethanolamine, sodium carbonate, sodium citrate and the like.

Examples of the suspending agent include surfactants such as stearyl triethanolamine, sodium lauryl sulfate, laurylaminopropionic acid, lecithin, benzalkonium chloride, benzetonium chloride, glycerin monostearate and the like; hydrophilic polymers such as poly(vinyl alcohol), polyvinylpyrrolidone, carboxymethylcellulose sodium, methylcellulose, hydroxymethylcellulose, hydroxyethylcellulose, hydroxypropylcellulose and the like; and the like.

Examples of the isotonic agent include glucose, D-sorbitol, sodium chloride, glycerin, D-mannitol and the like.

Examples of the buffering agent include buffer solutions such as phosphates, acetates, carbonates, citrates and the like.

Examples of the soothing agent include benzyl alcohol and the like.

Examples of the preservative include parahydroxybenzoates, chlorobutanol, benzyl alcohol, phenylethyl alcohol, dehydroacetic acid, sorbic acid and the like.

Examples of the antioxidant include sulfites, ascorbic acid, α-tocopherol and the like.

For the prophylaxis or treatment of various diseases, the compound of the present invention may also be used together with other medicaments. In the following, a medicament to be used when the compound of the present invention is used together with other drug is referred to as "the combination agent of the present invention".

For example, the compound of the present invention may be used together with the following drugs (concomitant drugs).
(1) therapeutic agent for diabetes
   insulin preparations (e.g., animal insulin preparations extracted from pancreas of bovine or swine; human insulin preparations genetically synthesized using *Escherichia coli* or yeast; zinc insulin; protamine zinc insulin; fragment or derivative of insulin (e.g., INS-1), oral insulin preparation), insulin sensitizers (e.g., pioglitazone or a salt thereof (preferably hydrochloride), rosiglitazone or a salt thereof (preferably maleate), Metaglidasen, AMG-131, Balaglitazone, MBX-2044, Rivoglitazone, Aleglitazar, Chiglitazar, Lobeglitazone, PLX-204, PN-2034, GFT-505, THR-0921, compound described in WO2007/013694, WO2007/018314, WO2008/093639 and WO2008/099794), α-glucosidase inhibitors (e.g., voglibose, acarbose, miglitol, emiglitate), biguanides (e.g., metformin or a salt thereof (preferably hydrochloride), buformin or a salt thereof (e.g., hydrochloride, fumarate, succinate)), insulin secretagogues [sulfonylurea (e.g., tolbutamide, glibenclamide, gliclazide, chlorpropamide, tolazamide, acetohexamide, glyclopyramide, glimepiride, glipizide, glybuzole), repaglinide, nateglinide, mitiglinide or calcium salt hydrate thereof], dipeptidyl-peptidase IV inhibitors (e.g., Alogliptin or a salt thereof (preferably benzoate), Vildagliptin, Sitagliptin, Saxagliptin, Teneligliptin, Linagliptin, Anagliptin, Melogliptin, Dutogliptin, PF-00734200, ALS2-0426, TA-6666, TS-021, KRP-104, Trelagliptin or a salt thereof (preferably succinic acid salt)), β3 agonists (e.g., N-5984), GPR40 agonist (e.g., fasiglifam, compound described in WO2004/041266, WO2004/106276, WO2005/063729, WO2005/063725, WO2005/087710, WO2005/095338, WO2007/013689 or WO2008/001931), GLP-1 receptor agonist [e.g., GLP-1, GLP-1MR agent, Liraglutide, Exenatide, AVE-0010, BIM-51077, Aib(8,35)hGLP-1(7,37)NH2, CJC-1131, Albiglutide], semaglutide, amylin agonist (e.g., pramlintide), phosphotyrosine phosphatase inhibitors (e.g., sodium vanadate), gluconeogenesis inhibitor (e.g., glycogen phosphorylase inhibitor, glucose-6-phosphatase inhibitor, glucagon antagonist, FBPase inhibitor), SGLT2 (sodium-glucose cotransporter 2) inhibitor (e.g., ipragliflozin, dapagliflozin, luseogliflozin, tofogliflozin, canagliflozin, empagliflozin, AVE2268, TS-033, YM543, TA-7284, Remogliflozin, ASP1941), SGLT1 inhibitor, 11β-hydroxysteroid dehydrogenase inhibitor (e.g., BVT-3498), adiponectin or agonist thereof, IKK inhibitor (e.g., AS-2868), leptin resistance improving drugs, somatostatin receptor agonists, glucokinase activators (e.g., Piragliatin, AZD1656, AZD6370, TTP-355, compound described in WO2006/112549, WO2007/028135, WO2008/047821, WO2008/050821, WO2008/136428 or WO2008/156757), GIP (Glucose-dependent insulinotropic peptide) and the like.
(2) therapeutic agent for diabetic complications
   aldose reductase inhibitors (e.g., Tolrestat, Epalrestat, Zopolrestat, Fidarestat, CT-112, ranirestat (AS-3201), Lidorestat), neurotrophic factors and increasing drugs thereof (e.g., NGF, NT-3, BDNF, neurotrophin production-secretion promoters described in WO01/14372 (e.g., 4-(4-chlorophenyl)-2-(2-methyl-1-imidazolyl)-5-[3-(2-methylphenoxy)propyl]oxazole), compound described in WO2004/039365)), nerve regeneration promoting drugs (e.g., Y-128), PKC inhibitors (e.g., ruboxistaurin mesylate), AGE inhibitors (e.g., ALT-946, pyratoxanthine, N-phenacylthiazolium bromide (ALT-766), ALT-711, EXO-226, Pyridorin, Pyridoxamine), GABA receptor agonists (e.g., gabapentin, Pregabalin), serotonin·noradrenaline reuptake inhibitors (e.g., duloxetine), sodium channel inhibitors (e.g., Lacosamide), active oxygen scavengers (e.g., thioctic acid), cerebral vasodilators (e.g., tiapuride, mexiletine), somatostatin receptor agonists (BIM23190), apoptosis signal regulating kinase-1 (ASK-1) inhibitors and the like.
(3) Therapeutic drug for cardiac failure
   (i) β receptor antagonists
      carvedilol, metoprolol, atenolol and the like.
   (ii) diuretic drug
      hydrochlorothiazide, spironolactone, furosemide, indapamide, bendrofluazide, cyclopenthiazide and the like.
   (iii) cardiotonic drug
      digoxin, dobutamine and the like.
   (iv) antialdosterone drug
      spironolactone, eplerenone and the like.
   (v) heart rate-lowering drug
      ivabradine and the like.
   (vi) intravenous cardiotonic injection
      h-ANP and the like.
   (vii) others
      relaxin and the like.
(4) Others
   (viii) Ca sensitizer
      MCC-135 and the like.
   (ix) Ca channel antagonist
      nifedipine, diltiazem, verapamil, lomerizine hydrochloride, amlodipine besylate and the like.
   (x) anti-platelet drug, anticoagulator
      heparin, aspirin, warfarin, dabigatran, rivaroxaban, apixaban, edoxaban and the like.
   (xi) HMG-CoA reductase inhibitor
      atorvastatin, simvastatin and the like.
   (xii) hypouricemic drug
      probenecid, allopurinol, febuxostat and the like.
   (xiii) α-blocker
      doxazosin and the like.
   (xiv) oral adsorptive agent
      kremezin and the like.
   (xv) therapeutic drug for hyperkalemia
      calcicol and the like.
   (xvi) therapeutic drug for hyperphosphatemia
      sevelamer, lanthanum carbonate and the like.
   (xvii) metabolic acidosis improving drug
      sodium bicarbonate and the like.
   (xviii) activity type vitamin

For combined use, the administration time of the compound of the present invention and the concomitant drug is not restricted, and the compound of the present invention or the concomitant drug can be administered to an administration subject simultaneously, or may be administered at different times. The dosage of the concomitant drug may be determined according to the dose clinically used, and can be appropriately selected depending on an administration subject, administration route, disease, combination and the like.

The administration form of the combined use is not particularly limited, and the compound of the present invention and a concomitant drug only need to be combined on administration. Examples of such administration mode include the following:
(1) administration of a single preparation obtained by simultaneously processing the compound of the present invention and the concomitant drug, (2) simultaneous administration of two kinds of preparations of the compound of the present invention and the concomitant drug, which have been separately produced, by the same administration route, (3) administration of two kinds of preparations of the compound of the present invention and the concomitant drug, which have been separately produced, by the same administration route in a staggered manner, (4) simultaneous administration of two kinds of preparations of the compound of the present invention and the concomitant drug, which have been separately produced, by different administration routes, (5) administration of two kinds of preparations of the compound of the present invention and the concomitant drug, which have been separately produced, by different administration routes in a staggered manner (e.g., administration in the order of the compound of the present invention and the concomitant drug, or in the reverse order) and the like.

The mixing ratio of the compound of the present invention and a concomitant drug in the combination agent of the present invention can be appropriately selected based on the subject of administration, administration route, disease and the like.

For example, while the content of the compound of the present invention in the combination agent of the present invention varies depending on the preparation form, it is generally about 0.01 - about 100 wt%, preferably about 0.1 - about 50 wt%, more preferably about 0.5 - about 20 wt%, of the whole preparation.

The content of the concomitant drug in the combination agent of the present invention varies depending on the preparation form, and generally about 0.01 to about 100% by weight, preferably about 0.1 to about 50% by weight, further preferably about 0.5 to about 20% by weight, of the whole preparation.

While the content of the additive such as a carrier and the like in the combination agent of the present invention varies depending on the form of a preparation, it is generally about 1 to about 99.99% by weight, preferably about 10 to about 90% by weight, based on the whole preparation.

When the compound of the present invention and the concomitant drug are separately prepared, the same content may be adopted.

Any amount of the concomitant drug can be adopted as long as the side effects do not cause a problem. The daily dosage in terms of the concomitant drug varies depending on the severity, age, sex, body weight, sensitivity difference of the subject, administration period, interval, and nature, pharmacology, kind of the pharmaceutical preparation, kind of effective ingredient, and the like, and not particularly restricted, and the amount of a drug is, in the case of oral administration for example, generally about 0.001 to about 2000 mg, preferably about 0.01 to about 500 mg, further preferably about 0.1 to about 100 mg, per 1 kg of a mammal and this is generally administered once to 4-times, divided in a day.

When the combination agent of the present invention is administered, the compound of the present invention and the concomitant drug can be administered simultaneously, or may be administered in a staggered manner. When administered at a time interval, the interval varies depending on the effective ingredient, dosage form and administration method, and, for example, when the concomitant drug is administered first, a method in which the compound of the present invention is administered within time range of from 1 minute to 3 days, preferably from 10 minutes to 1 day, more preferably from 15 minutes to 1 hour, after administration of the concomitant drug is an example. When the compound of the present invention is administered first, a method in which the concomitant drug is administered within time range of from 1 minute to 1 day, preferably from 10 minutes to 6 hours, more preferably from 15 minutes to 1 hour after administration of the compound of the present invention is an example.

### Examples

The present invention is explained in detail in the following by referring to Examples, Experimental Examples and Formulation Examples, which are not to be construed as limitative, and the invention may be changed within the scope of the present invention.

In the following Examples, the "room temperature" generally means about 10°C to about 35°C. The ratios indicated for mixed solvents are volume mixing ratios, unless otherwise specified. % means wt%, unless otherwise specified.

The elution by column chromatography in the Examples was performed under observation by TLC (thin layer chromatography), unless otherwise specified. In TLC observation, 60 F₂₅₄ manufactured by Merck was used as TLC plate and, as the eluent, the solvent used as an elution solvent in column chromatography was used. For detection, UV detector was employed. In silica gel column chromatography, "NH" means use of aminopropylsilane-bound silica gel and "Diol" means use of 3-(2,3-dihydroxypropoxy)propylsilane-bound silica gel. In preparative HPLC (high performance liquid chromatography), "C18" means use of octadecyl-bound silica gel. The ratios of elution solvents are volume mixing ratios, unless otherwise specified.

For the analysis of ¹H NMR, ACD/SpecManager (trade name) software and the like were used. Peaks of a hydroxy group, an amino group and the like, which having very mild proton peaks, are not described sometimes.

MS was measured by LC/MS. As an ionization method, ESI method or APCI method was used. The data indicates those found. Generally, a molecular ion peak is observed but it may be observed as a fragment ion. In the case of a salt, a molecular ion peak or fragment ion peak of free form is generally observed.

The unit of sample concentration (c) in optical rotation ([α]_{D}) is g/100 mL.

As the elemental analysis values (Anal.), calculated values (Calcd) and measured values (Found) are described.

In the Examples, the peak by a powder X-ray diffraction means a peak measured at room temperature using Ultima IV (Rigaku Corporation, Japan) with a Cu-Kα ray as a ray source. The measurement conditions are as follows.
Electric pressure/Electric current: 40 kV/50 mA
Scan speed: 6 degree/min
Scan range of 2 Theta: 2-35 degree

The crystallinity by powder X-ray diffraction in the Examples was calculated by the Hermans method.

The X-ray crystal structure analysis in the Examples included measurement of diffraction data by XtaLAB P200 manufactured by Rigaku Corporation. The initial phase was determined by the direct method (SIR2008 (Burla, M. C.; Caliandro, R.; Camalli, M.; Carrozzini, B.; Cascarano, G. L.; De Caro, L.; Giacovazzo, C.; Polidori, G.; Siliqi, D.; Spagna, R. SIR2008: Program for the Solution of Crystal Structures from X-ray Data; CNR Institute of Crystallography: Bari, Italy, 2007.)) and the structure was refined using the full-matrix least-squares method (SHELXL-2014/7 (Sheldrick, G.M. Acta Cryst. A 2008, 64, 112-122.)). An anisotropic temperature factor was applied to non-hydrogen atoms and an isotropic temperature factor was applied to a hydrogen atom.

In Examples, the following abbreviations are used.
mp: melting point
MS: mass spectrum
M: mol concentration
N: normality
CDCl₃: deuterochloroform
DMSO-d₆: deuterodimethyl sulfoxide
CD₃OD: deuterated methanol
¹H NMR: proton nuclear magnetic resonance
LC/MS: liquid chromatograph mass spectrometer
ESI: electrospray ionization
APCI: atomospheric pressure chemical ionization
TFA: trifluoroacetic acid

### Comparative Example 1

### (1r,4r)-4-(2,4-dichlorophenoxy)-2'-oxo-1',2'-dihydrospiro[cyclohexane-1,3'-indole]-5'-carboxylic acid [0143]

### A) dimethyl 2',4-dioxo-1',2'-dihydrospiro[cyclohexane-1,3'-indole]-3,5'-dicarboxylate

To a mixture of methyl 2-oxoindoline-5-carboxylate (57.4 g), potassium tert-butoxide (1.68 g) and dimethyl sulfoxide (300 ml) was added methyl acrylate (81 ml) at 40°C over 1 hr. At that time, the internal temperature rose to 60°C. The reaction mixture was stirred under a nitrogen atmosphere at 60°C for 30 min, and potassium tert-butoxide (20.3 g) was added 4 times (total amount 81 g) at 10 min intervals. The reaction mixture was stirred under a nitrogen atmosphere at 60°C for 1 hr, neutralized with 6N hydrochloric acid at 10°C-15°C, and extracted with ethyl acetate. The organic layer was separated, washed with water, saturated aqueous sodium hydrogen carbonate solution and saturated brine, dried over anhydrous magnesium sulfate and concentrated under reduced pressure. The residue was washed with ethyl acetate-hexane (1:4) to give the title compound (72.0 g).
MS: [M-H]⁻ 330.0.

### B) methyl 2',4-dioxo-1',2'-dihydrospiro[cyclohexane-1,3'-indole]-5'-carboxylate

A mixture of dimethyl 2',4-dioxo-1',2'-dihydrospiro[cyclohexane-1,3'-indole]-3,5'-dicarboxylate (105 g), water (5.71 ml), sodium chloride (18.5 g) and dimethyl sulfoxide (400 ml) was stirred at 150°C for 5 hr. The reaction mixture was poured into water, and the mixture was extracted with ethyl acetate. The organic layer was separated, washed with water and saturated brine, dried over anhydrous magnesium sulfate and concentrated under reduced pressure. The residue was washed with ethyl acetate to give the title compound (55.2 g).
MS: [M-H]⁻ 272.0.

### C) methyl (1s,4s)-4-hydroxy-2'-oxo-1',2'-dihydrospiro[cyclohexane-1,3'-indole]-5'-carboxylate (I) and methyl (1r,4r)-4-hydroxy-2'-oxo-1',2'-dihydrospiro[cyclohexane-1,3'-indole]-5'-carboxylate (II)

To a solution of methyl 2',4-dioxo-1',2'-dihydrospiro[cyclohexane-1,3'-indole]-5'-carboxylate (3.05 g) in tetrahydrofuran (75 ml) was added sodium borohydride (0.844 g) at 0°C, and the mixture was stirred at room temperature overnight. To the reaction mixture was added 1N hydrochloric acid at room temperature, and the mixture was extracted with ethyl acetate. The organic layer was separated, washed with water and saturated brine, dried over anhydrous magnesium sulfate and concentrated under reduced pressure. The residue was purified by silica gel column chromatography (ethyl acetate/hexane) to give the title compound (I) (1.35 g) and the title compound (II) (110 mg).
title compound (I)
   ¹H NMR (400 MHz, DMSO-d₆) 61.60-1.82 (6H, m), 1.87-2.03 (2H, m), 3.71-3.78 (1H, m), 3.81 (3H, s), 4.59 (1H, d, J = 4.0 Hz), 6.92 (1H, d, J = 8.8 Hz), 7.82-7.86 (2H, m), 10.65 (1H, d, J = 1.0 Hz).; MS: [M-H]⁻ 274.0.
title compound (II)
   1H NMR (400 MHz, DMSO-d₆) δ1.56-1.73 (6H, m), 1.98-2.02 (2H, m), 3.68 (1H, brs), 3.83 (3H, s), 4.83 (1H, d, J = 4.1 Hz), 6.97 (1H, d, J = 8.3 Hz), 7.88 (1H, dd, J = 8.2, 1.3 Hz), 8.02 (1H, d, J = 1.0 Hz), 10.78 (1H, brs).; MS: [M-H]⁻ 274.0.

### D) methyl (1r,4r)-4-(2,4-dichlorophenoxy)-2'-oxo-1',2'-dihydrospiro [cyclohexane-1, 3'-indole]-5'-carboxylate

To a mixture of diisopropyl azodicarboxylate (1.9 mol/l toluene solution, 2.87 ml), triphenylphosphine (1.43 g) and tetrahydrofuran (20 ml) was added 2,4-dichlorophenol (0.711 g) at room temperature, and a solution of methyl (1s,4s)-4-hydroxy-2'-oxo-1',2'-dihydrospiro[cyclohexane-1,3'-indole]-5'-carboxylate (1.00 g) in tetrahydrofuran (20 ml) was added. After stirring at room temperature overnight, water was added to the mixture and the mixture was extracted with ethyl acetate. The organic layer was separated, washed with water and saturated brine, dried over anhydrous magnesium sulfate and concentrated under reduced pressure. The residue was purified by silica gel column chromatography (ethyl acetate/hexane) to give the title compound (1.37 g) as a crude purified product. MS: [M-H]⁻ 418.1.

### E) (1r,4r)-4-(2,4-dichlorophenoxy)-2'-oxo-1',2'-dihydrospiro[cyclohexane-1,3'-indole]-5'-carboxylic acid

To a mixture of methyl (1r,4r)-4-(2,4-dichlorophenoxy)-2'-oxo-1',2'-dihydrospiro[cyclohexane-1,3'-indole]-5'-carboxylate (1.37 g), tetrahydrofuran (15 ml) and methanol (15 ml) was added 2N aqueous sodium hydroxide solution (16.3 ml) at room temperature. The mixture was stirred at 50°C overnight and concentrated under reduced pressure. The residue was diluted with water, the aqueous layer was washed with ethyl acetate, neutralized with 6N hydrochloric acid, and the mixture was extracted with ethyl acetate. The organic layer was separated, washed with water and saturated brine, dried over anhydrous magnesium sulfate and concentrated under reduced pressure. The residue was purified by silica gel column chromatography (ethyl acetate/hexane) and the obtained solid was crystallized from methanol/water to give the title compound (320 mg).
1H NMR (400 MHz, DMSO-d₆) δ1.69 (2H, dd, J = 11.1, 6.2 Hz), 1.82-1.98 (4H, m), 2.23-2.37 (2H, m), 4.85 (1H, brs), 6.94 (1H, d, J = 8.0 Hz), 7.37 (2H, s),7.62 (1H, s), 7.85 (1H, d, J = 8.2 Hz), 7.89 (1H, s), 10.72 (1H, s), 12.68 (1H, brs).

### Example 1

### (1s,4s)-4-((3,5-dichloropyridin-2-yl)oxy)-2'-oxo-1',2'-dihydrospiro[cyclohexane-1,3'-indole]-5'-carboxylic acid

### A) methyl (1s,4s)-4-hydroxy-2'-oxo-1',2'-dihydrospiro [cyclohexane-1, 3'-indole]-5'-carboxylate

To a mixture of methyl 2',4-dioxo-1',2'-dihydrospiro[cyclohexane-1,3'-indole]-5'-carboxylate (26 g) and tetrahydrofuran (300 ml) was added borane-tetrahydrofuran complex (1 mol/l tetrahydrofuran solution, 95 ml) at 0°C, and the mixture was stirred at the same temperature for 20 min. To the reaction mixture were added water at 0°C and 1N hydrochloric acid (8 ml), and the mixture was extracted with ethyl acetate. The organic layer was separated, washed with water and saturated brine, dried over anhydrous magnesium sulfate and concentrated under reduced pressure. The residue was purified by silica gel column chromatography (ethyl acetate/hexane) to give the title compound (14.6 g).
¹H NMR (300 MHz, DMSO-d₆) δ1.58-1.84 (6H, m), 1.86-2.06 (2H, m), 3.65-3.87 (4H, m), 4.60 (1H, d, J = 3.9 Hz), 6.92 (1H, dd, J = 8.6, 1.3 Hz), 7.73-7.90 (2H, m), 10.65 (1H, s).; MS: [M-H]⁻ 274.0.

### B) methyl (1s,4s)-4-((3,5-dichloropyridin-2-yl)oxy)-2'-oxo-1',2'-dihydrospiro[cyclohexane-1,3'-indole]-5'-carboxylate

To a suspension of sodium hydride (60% in oil, 3.71 g) and tetrahydrofuran (200 ml) was added methyl (1s,4s)-4-hydroxy-2'-oxo-1',2'-dihydrospiro[cyclohexane-1,3'-indole]-5'-carboxylate (8.50 g) at 0°C, and the mixture was stirred at the same temperature for 30 min. After stirring, 3,5-dichloro-2-fluoropyridine (6.15 g) was added. After stirring at room temperature overnight, water was added to the reaction mixture and the mixture was extracted with ethyl acetate. The organic layer was separated, washed with water and saturated brine, dried over anhydrous magnesium sulfate and concentrated under reduced pressure. The residue was purified by silica gel column chromatography (ethyl acetate/hexane) to give the title compound (11.9 g).
MS: [M+H]⁺ 421.1.

### C) (1s,4s)-4-((3,5-dichloropyridin-2-yl)oxy)-2'-oxo-1',2'-dihydrospiro[cyclohexane-1,3'-indole]-5'-carboxylic acid

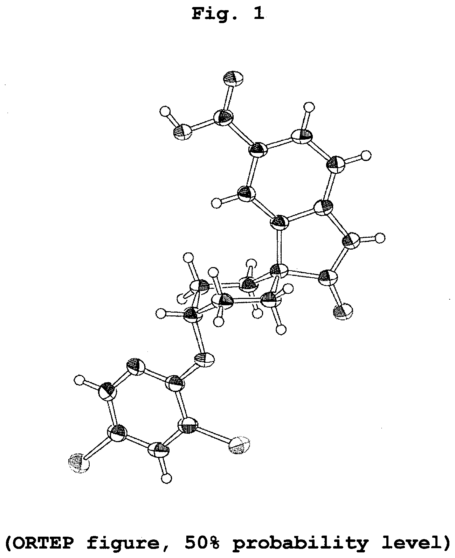

To a mixture of methyl (1s,4s)-4-((3,5-dichloropyridin-2-yl)oxy)-2'-oxo-1',2'-dihydrospiro[cyclohexane-1,3'-indole]-5'-carboxylate (100 g), methanol (500 ml) and tetrahydrofuran (500 ml) was added 2N aqueous sodium hydroxide solution (475 ml), and the mixture was stirred at 55°C for 5 hr. To the reaction mixture was added 2N hydrochloric acid (475 ml) at room temperature, and the mixture was stirred at 15°C-25°C for 1 hr. The resulting precipitate was collected by filtration and washed with methanol/water (1:1, 200 ml) to give a white solid. The obtained solid was dissolved in dimethyl sulfoxide (300 ml) at 80°C, insoluble materials were removed by filtration, and dimethyl sulfoxide (60 ml) was added. Ethanol (630 ml) was added to the filtrate while maintaining at 50°C and water (180 ml) was added at 50°C-60°C. The mixture was stirred at the same temperature for 30 min. After stirring, the mixture was slowly cooled to room temperature, further ice-cooled and stirred. The mixture was filtered, and the filtrate was washed with ethanol-water (1:1, 200 ml) to give the title compound (80 g). The data of the crystal of the title compound is shown in Table 4 and the molecule structure obtained by single crystal X-ray structure analysis is shown in Fig. 1.
¹H NMR (300 MHz, DMSO-d₆) δ1.78-2.09 (6H, m), 2.18-2.35 (2H, m), 5.28-5.40 (1H, m), 6.92 (1H, d, J = 8.1 Hz), 7.84 (1H, d, J = 8.1 Hz), 7.95 (1H, s), 8.15-8.26 (2H, m), 10.72 (1H, s), 12.64 (1H, brs).; MS: [M-H] ⁻ 404.9.; Anal. Calcd for C₁₉H₁₆N₂O₄Cl₂: C, 56.04; H, 3.96; N, 6.88. Found: C, 55.67; H, 4.12; N, 7.10.; mp 302 °C.

**[Table 4]**

| | |
|---|---|
| molecular formula | C₁₉H₁₆Cl₂N₂O₄ |
| molecular weight | 407.25 |
| color of crystal, crystal habit | colorless, plate |
| temperature (K) | 100 |
| crystal size (mm) | 0.151 x 0.122 x 0.068 |
| crystal system | monoclinic |
| space group | I2/a |
| lattice constant (Å, °) | a = 17.5416(4) |
| | b = 7.1049(3) |
| | c = 31.6985(9) |
| | α = γ = 90°, β = 90.346(2)° |
| volume (Å³) | 3950.6(2) |
| Z | 8 |
| density (Calculated) (g/cm³) | 1.369 |
| radiation | Cu-Kα (1.5419 Å) |
| absorption coefficient (mm⁻¹) | 0.319 |
| goodness of fit on F² | 1.060 |
| R₁ [I>2σ(I)] | 0.055 |
| wR₂ (all data) | 0.168 |

### Comparative Example 2

### (1s,4s)-4-((3,5-dichloropyridin-2-yl)oxy)-2'-oxo-1',2'-dihydrospiro[cyclohexane-1,3'-pyrrolo[3,2-b]pyridine]-5'-carboxylic acid

### A) methyl (1r,4r)-4-hydroxy-2'-oxo-1',2'-dihydrospiro[cyclohexane-1,3'-pyrrolo[3,2-b]pyridine]-5'-carboxylate

To a mixture of lithium tri-sec-butylborohydride (1.02 M tetrahydrofuran solution, 7.15 ml) and tetrahydrofuran (20 ml) was added a solution of methyl 2',4-dioxo-1',2'-dihydrospiro[cyclohexane-1,3'-pyrrolo[3,2-b]pyridine]-5'-carboxylate (1.0 g) in tetrahydrofuran (20 ml) at -60°C to - 70°C. The reaction mixture was stirred at -70°C for 1 hr, saturated aqueous ammonium chloride solution and saturated brine were added at -70°C, and the mixture was extracted with ethyl acetate. The organic layer was separated, washed with water and saturated brine, dried over anhydrous magnesium sulfate and concentrated under reduced pressure. The residue was washed with ethanol-diethyl ether to give the title compound (740 mg).

¹H NMR (300 MHz, DMSO-d₆) δ1.49-1.72 (2H, m), 1.73-2.09 (6H, m), 3.64-3.77 (1H, m), 3.86 (3H, s), 4.67 (1H, d, J = 2.8 Hz), 7.22-7.34 (1H, m), 7.87-7.99 (1H, m), 10.45-11.30 (1H, m).; MS: [M+H]⁺ 277.3.

### B) 1'-tert-butyl 5'-methyl (1r,4r)-4-hydroxy-2'-oxospiro[cyclohexane-1,3'-pyrrolo[3,2-b]pyridine]-1',5'(2'H)-dicarboxylate

A mixture of di-tert-butyl dicarbonate (9.66 g), sodium hydrogen carbonate (4.96 g), methyl (1r,4r)-4-hydroxy-2'-oxo-1',2'-dihydrospiro[cyclohexane-1,3'-pyrrolo[3,2-b]pyridine]-5'-carboxylate (8.15 g) and tetrahydrofuran (300 ml) was stirred at room temperature for 24 hr, tetrahydrofuran (200 ml) was added and the mixture was further stirred for 16 hr. The reaction mixture was concentrated under reduced pressure, water was added and the mixture was extra cted with ethyl acetate. The organic layer was sepa rated, washed with water and saturated brine, dried over anhydrous magnesium sulfate and concentrated under reduced pressure. The residue was washed with hexane to give the title compound (10.2 g).
MS: [M+H]⁺ 377.3.

### C) 1'-tert-butyl 5'-methyl (1s,4s)-4-((3,5-dichloropyridin-2-yl)oxy)-2'-oxospiro[cyclohexane-1,3'-pyrrolo[3,2-b]pyridine]-1',5' (2'H)-dicarboxylate

A solution of diethyl azodicarboxylate (5.49 ml) in tetrahydrofuran (10 ml) was added to a suspension of 1'-tert-butyl 5'-methyl (1r,4r)-4-hydroxy-2'-oxospiro[cyclohexane-1,3'-pyrrolo[3,2-b]pyridine]-1',5' (2'H)-dicarboxylate (8.70 g), 3,5-dichloropyridin-2-ol (5.69 g), triphenylphosphine (9.09 g) in tetrahydrofuran (150 ml) at 0°C, and the mixture was stirred at room temperature for 4 hr. Water was added to the reaction mixture and the mixture was extracted with ethyl acetate. The organic layer was separated, washed with water and saturated brine, dried over anhydrous magnesium sulfate and concentrated under reduced pressure. The residue was purified by silica gel column chromatography (ethyl acetate/hexane), and the obtained solid was washed with hexane to give the title compound (7.80 g).
MS: [M+H]⁺ 522.2.

### D) methyl (1s,4s)-4-((3,5-dichloropyridin-2-yl)oxy)-2'-oxo-1',2'-dihydrospiro[cyclohexane-1,3'-pyrrolo[3,2-b]pyridine]-5'-carboxylate

A mixture of 1'-tert-butyl 5'-methyl (1s,4s)-4-((3,5-dichloropyridin-2-yl)oxy)-2'-oxospiro[cyclohexane-1,3'-pyrrolo[3,2-b]pyridine]-1',5'(2'H)-dicarboxylate (10.3 g) and 10% hydrochloric acid methanol solution (500 ml) was stirred at 50°C for 16 hr and concentrated under reduced pressure. To the residue was added saturated aqueous sodium hydrogen carbonate solution and the mixture was extracted with ethyl acetate-tetrahydrofuran. The organic layer was separated, washed with saturated brine, dried over anhydrous magnesium sulfate and concentrated under reduced pressure. The residue was washed with hexane to give the title compound (8.26 g).
¹H NMR (300 MHz, DMSO-d₆) δ1.61-1.84 (2H, m), 1.94-2.21 (4H, m), 2.25-2.40 (2H, m), 3.86 (3H, s), 5.27-5.44 (1H, m), 7.31 (1H, d, J = 8.2 Hz), 7.90-8.01 (1H, m), 8.17-8.20 (1H, m), 8.21-8.29 (1H, m), 10.91-11.06 (1H, m).

### E) (1s,4s)-4-((3,5-dichloropyridin-2-yl)oxy)-2'-oxo-1',2'-dihydrospiro[cyclohexane-1,3'-pyrrolo[3,2-b]pyridine]-5'-carboxylic acid

A mixture of methyl (1s,4s)-4-((3,5-dichloropyridin-2-yl)oxy)-2'-oxo-1',2'-dihydrospiro[cyclohexane-1,3'-pyrrolo[3,2-b]pyridine]-5'-carboxylate (14.8 g), 1N aqueous sodium hydroxide solution (150 ml), methanol (150 ml) and tetrahydrofuran (150 ml) was stirred at 60°C for 5 hr, 1N hydrochloric acid (150 ml) and water were added and the mixture was stirred at room temperature for 1 hr. The resulting precipitate was collected by filtration and the obtained solid was crystallized from ethanol/water to give the title compound (12.7 g).
¹H NMR (300 MHz, DMSO-d₆) δ1.61-1.82 (2H, m), 1.94-2.20 (4H, m), 2.27-2.46 (2H, m), 5.35 (1H, d, J = 3.2 Hz), 7.31 (1H, s), 7.95 (1H, d, J = 8.2 Hz), 8.17-8.20 (1H, m), 8.21-8.25 (1H, m), 10.68-11.32 (1H, m), 12.31-13.10 (1H, m).; MS: [M-H]⁻ 405.9.; Anal. Calcd for C₁₈H₁₅Cl₂N₃O₄, C; 52.96. H; 3.70, N; 10.29. Found. C; 52.97, H; 3.62, N; 10.31.; mp 282-283 °C.

**[Table 5]**

| Ex. No. | IUPAC name | structural formula | MS |
|---|---|---|---|
| Comp. Ex. 1 | (1r,4r)-4-(2,4-dichlorophenoxy)-2'-oxo-1',2'-dihydrospiro[cyclohexane-1,3'-indole]-5'-carboxylic acid | 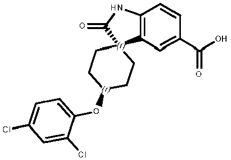 | 404 |
| Ex. 1 | (1s,4s)-4-((3,5-dichloropyridin-2-yl)oxy)-2'-oxo-1',2'-dihydrospiro[cyclohexane-1,3'-indole]-5'-carboxylic acid | 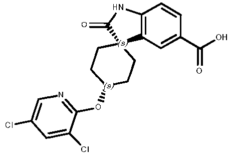 | 404.9 |
| Comp. Ex. 2 | (1s,4s)-4-((3,5-dichloropyridin-2-yl)oxy)-2'-oxo-1',2'-dihydrospiro[cyclohexane-1,3'-pyrrolo[3,2-b]pyridine]-5'-carboxylic acid | 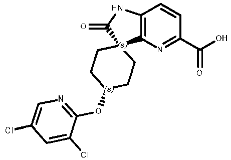 | 405.9 |

### Experimental Example 1

### IP6K enzyme inhibitory assay

### Method

Human IP6K1 and human IP6K2 enzymes were prepared by transducing human IP6K1 (1-441) gene or human IP6K2 (1-426) gene into Sf-9 insect cells and purifying by GST affinity column. Human IP6K3 enzyme was prepared by transducing human IP6K3 (1-410) gene into BL21 (DE3) Escherichia coli and purifying by Ni-NTA (nickel-nitrilotriacetic acid) affinity column. Rat IP6K3 and mouse IP6K3 enzymes were prepared by transducing rat IP6K3 (1-401) gene or mouse IP6K3 (1-396) gene into FreeStyleTM293 cells and purifying by GST affinity column. The enzymes were used after preservation at -70°C. The IP6K enzyme inhibitory activity of the test compound was measured by the following experiment method and using ADP-GloTM Kinase Assay (manufactured by Promega). A test compound diluted with an assay buffer (20 mM HEPES (pH 7.5), 6 mM MgCl₂, 0.01% Tween-20, 1 mM DTT) was first added by 2 µL to a 384 well plate. Then, ATP.IP6 substrate mixed solution (human IP6K1, IP6K2, IP6K3: ATP.IP6 = 135 µM.45 µM, rat IP6K3: ATP.IP6 = 300 µM. 4.5 µM, mouse IP6K3: ATP.IP6 = 150 µM•15 µM) was added by 2 µL. The IP6K enzyme solution diluted with the assay buffer was added by 2 µL to start the enzyme reaction. Incubation was performed at room temperature for a given time (120 min for rat IP6K3 alone, 60 min for others). After incubation at room temperature, ADP-Glo solution prepared according to the protocol of Promega was added by 3 µL to the 384 well plate and reacted at room temperature for 30 min. Thereafter, Kinase-Detection solution was added by 6 µL to the 384 well plate and reacted at room temperature for 60 min. After the reaction, the luminescence intensity was measured by a plate reader Envision (manufactured by PerkinElmer). The inhibitory activity of each compound was calculated as a relative activity value to luminescence intensity of the well without enzyme as 100% inhibition. The results are shown in Table 6.

**[Table 6]**

| | IP6K inhibitory activity of Example compounds | | |
|---|---|---|---|
| | ADP-Glo method inhibitory rate (%) at compound concentration 0.1 µM | | |
| Example | human IP6K1 | human IP6K2 | human IP6K3 |
| Comp. Ex. 1 | 100 | 100 | 99 |
| Ex. 1 | 98 | 101 | 94 |
| Comp. Ex. 2 | 101 | 101 | 110 |

### Experimental Example 2 (not according to the invention)

### tissue IP7 lowering effect in rat

### Method

Vehicle (0.5% methyl cellulose) and compound 97 (compound of Comparative Example 2) were orally administered at 1, 3, 10 mg/kg to 20-week-old male Zucker fatty rats (obtained from TAKEDA RABICS), and the IP7 level of the liver, skeletal muscle (soleus muscle) and fat (epididymal fat) was measured 6 and 24 hr later.

### IP7 measurement method

### Extraction 1

To cryopreserved tissues was added 1.0 mL of an aqueous solution of 1N sodium hydroxide/0.2 M ethylenediaminetetraacetic acid per 100 mg of the tissue, pulverized with zirconia beads under 4°C condition and stood on ice for 1 hr. 0.1 mL of 25% acetic acid was added per 0.4 mL of the pulverized liquid, and they were mixed and centrifuged (15000 rpm, 5 min, 4°C). Then, the supernatant was subjected to ultrafiltration (13000 rpm, 5 min, 4°C; microcon YM-3 membrane centrifugal filter unit (Millipore)). The passage fraction was recovered, equal amounts of 1.5% acetic acid/1.5% aqueous octylamine solution were added and the mixture was stirred by Vortex and 0.05 mL was injected into LC/MS/MS.

### Extraction 2

To cryopreserved tissues was added 0.3 mL of 3.6% perchloric acid per 100 mg of the tissue and the mixture was pulverized with zirconia beads under 4°C condition. Then, 0.1 mL of 30% aqueous potassium chloride solution was added per 0.3 mL of 3.6% perchloric acid added, and they were mixed and centrifuged (15000 rpm, 5 min, 4°C). Then, the supernatant was subjected to ultrafiltration (13000 rpm, 5 min, 4°C; microcon YM-3 membrane centrifugal filter unit (Millipore)). The passage fraction was recovered, equal amounts of 1.5% acetic acid/1.5% aqueous octylamine solution were added and the mixture was stirred by Vortex and 0.01 mL was injected into LC/MS/MS.

Samples subjected to a protein removal treatment by the methods of extraction 1 and extraction 2 were measured by the LC/MS/MS system containing Nexera UHPLC system (Shimadzu Corporation) and QTRAP (registered trade mark) 5500 mass spectrometer system (SCIEX) in combination. The column used was Triat PEEK column (30×2.1 mm, 3 µm, GL Sciences Inc.), solvent A used was an aqueous solution of 0.1% ammonia/0.01% octylamine/10 µM ethylenediaminetetraacetic acid and solvent B used was 0.01% ammonia/0.002% octylamine/methanol solution. The flow rate was set to 0.7 mL per min, the column temperature was set to 50°C, the ratio of solvent B to solvent A was set to the following and inositolpolyphosphoric acid was separated on chromatography. After injecting the sample, the measurement was performed at 2% isocratic for 0-0.5 min, 2% to 50% gradient for 0.5-2 min, 50% to 90% gradient for 2-5 min, 90% isocratic for 5-6 min and 2% isocratic for 6-8 min. The eluate was ionized in the negative ion mode by mass spectrometry and ion intensity of the measurement target molecule was measured under the MRM conditions in Table 7. The conditions of the instrument were ion spray voltage 4500 V, turbo probe temperature 400°C, heat gas 40 L per min, nitrogen 12 L per min as curtain gas, multiplier 2100 V, declustering potential -80 V, collision cell exit potential -11V.

**[Table 7]**

| measurement target molecule | Precursor ion (m/z) | Product ion (m/z) | CE (V) | elution time (min) |
|---|---|---|---|---|
| IP5 | 579 | 481 | -38 | 3.89 |
| IP6 | 659 | 561 | -35 | 4.01 |
| IP7 | 739 | 641 | -35 | 4.08 |
| | 739 | 159 | -85 | 4.08 |
| IP8 | 819 | 721 | -38 | 4.11 |

| | | | | |
|---|---|---|---|---|
| CE; collision energy | | | | |

The area value of the mass chromatogram obtained by the measurement was calculated by MultiQuant ver 3.0(SCIEX).

### Results

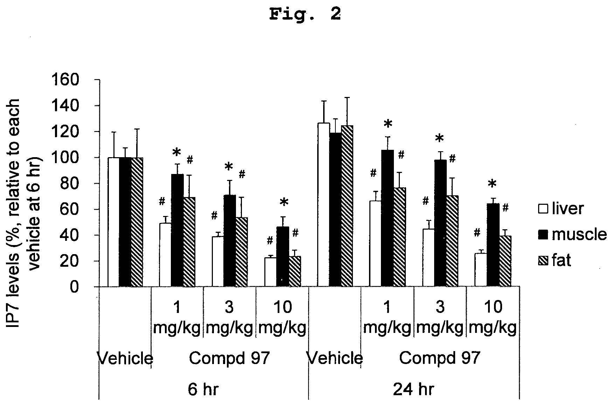

The results are shown in Fig. 2. Compound 97 showed a dose-dependent and sustained decrease in the IP7 level of the liver, muscle and fat.
*p<0.025 vs vehicle-treated rat by one-tailed Williams' test #p<0.025 vs vehicle-treated rat by one-tailed Shirley Williams test

### Experimental Example 3 (not according to the invention)

### Non-fasting hyperglycemic lowering effect in diabetes rat

### Method

Vehicle (0.5% methyl cellulose), compound 97 at 3 or 10 mg/kg and metformin at 150 mg/kg were orally administered to 12-week-old male Zucker fatty rats having equalized glycosylated hemoglobin, body weight and non-fasting blood glucose (n=6, Zucker lean rats (n=4) obtained from CHARLES RIVER LABORATORIES JAPAN, INC. as normal control) and the blood glucose level was measured for 24 hr.

### Results

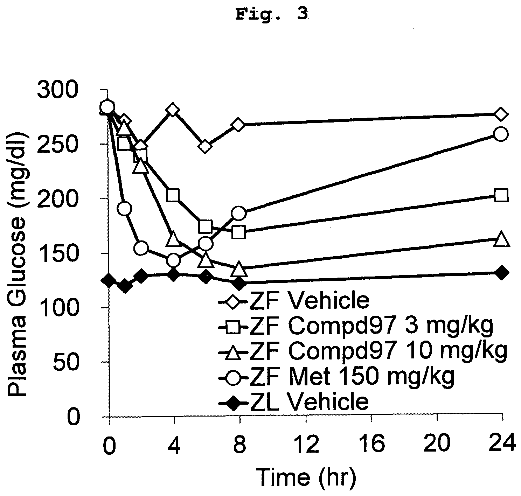

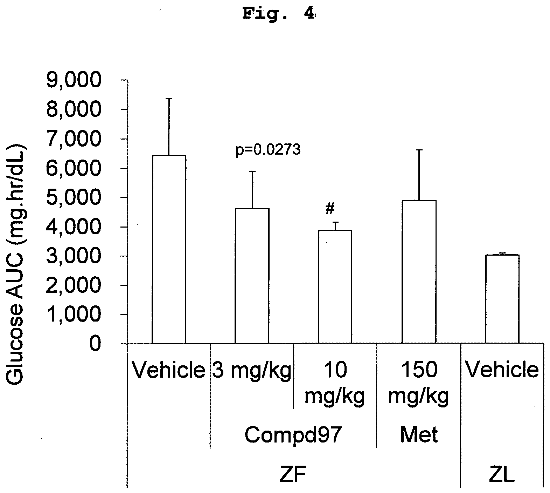

The results are shown in Fig. 3 and Fig. 4. Compound 97 showed a sustained lowering effect on hyperglycemia during non-fasting and a significant hypoglycemic action (decrease in glucose AUC up to 24 hr) was confirmed in compound 97 (10 mg/kg) administration group.
Mean±SD, n=6(ZF) and 4(ZL)
ZF: Zucker fatty
ZL: Zucker lean
Compd 97: compound 97
Met: Metformin
#p<0.025 vs vehicle-treated ZF rat by Shirley Williams test

### Experimental Example 4

### Glucose tolerance improving effect and decrease in tissue IP7 in rat with impaired glucose tolerance

### Method

Vehicle (0.5% methyl cellulose) or compound 9 (compound of Example 1) at 0.3, 3, and 30 mg/kg was orally administered to 17-week-old male Zucker fatty rats (n=6, obtained from Takeda RABICS) ) having equalized glycosylated hemoglobin and body weight and the rats were fasted for 16 hr. Then, fasting blood glucose was measured, glucose was orally loaded (2 g/kg) and the blood glucose level was measured for 120 min. After blood sample collection for 120 min, tissues (soleus muscle and liver) were promptly collected and the content of IP7 in the tissue was measured.

### Results

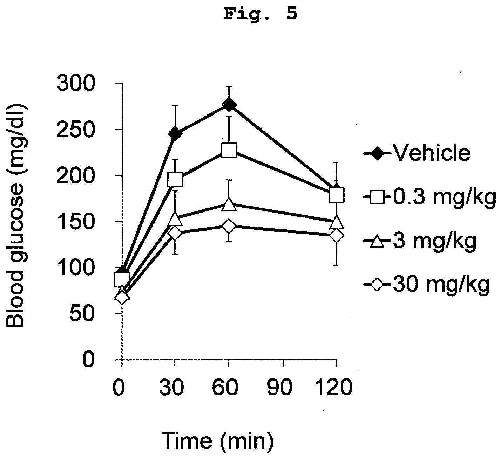

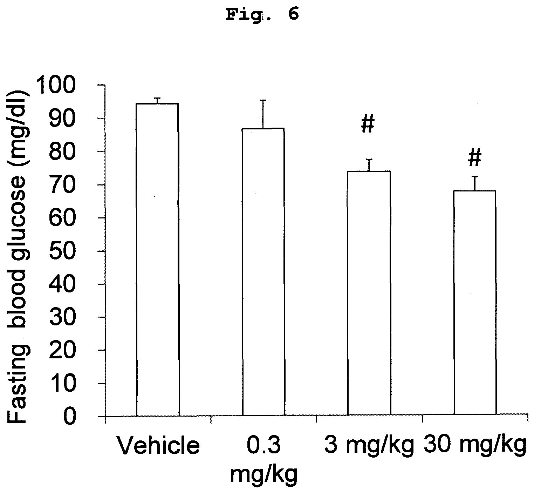

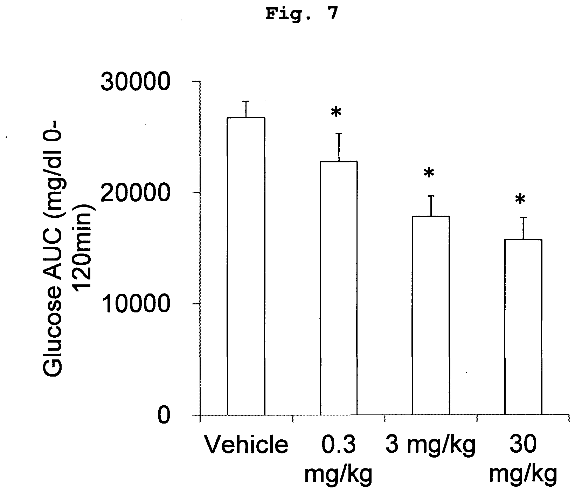

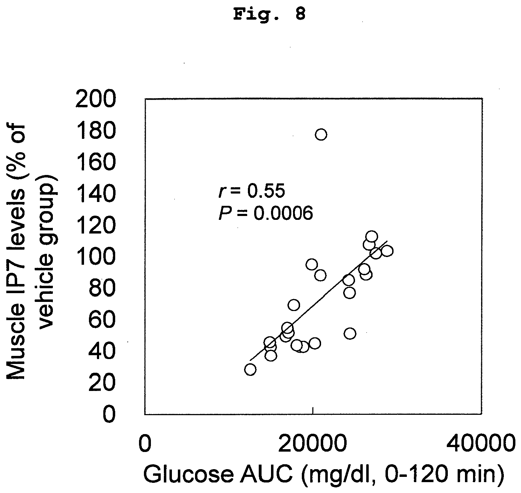

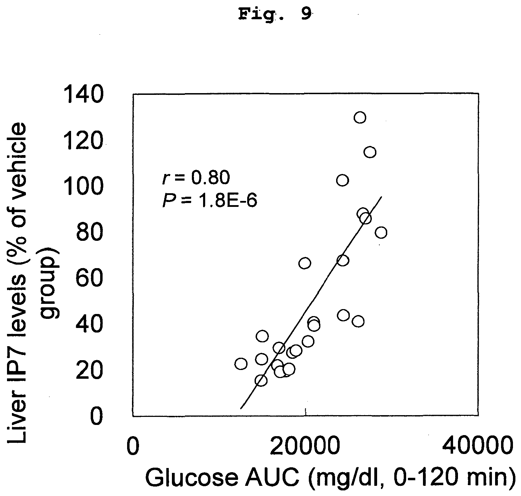

The results are shown in Fig. 5 - Fig. 9. In rats with impaired glucose tolerance, administration of compound 9 decreased fasting blood glucose from 3 mg/kg and improved glucose tolerance from 0.3 mg/kg (glucose AUC in 0-120 min). The glucose tolerance-improving effect (glucose AUC in 0-120 min) correlated well with the decrease in IP7 in the muscle (soleus muscle) and liver.
Mean±SD, n=6.
*p<0.025 vs vehicle-treated ZF rat by one-tailed Williams' test #p<0.025 vs vehicle-treated ZF rat by one-tailed Shirley Williams test

### Experimental Example 5

### Life prolonging effect in cardiac failure model mouse

### Method

Vehicle (0.5% methylcellulose solution), compound 97 (10 mg/kg) and compound 9 (10 mg/kg) were orally administered once per day (n=21) to male CSQ TG mouse (6-week-old) as adilated cardiomyopathy model. As a normal control, non-Tg mouse (n=5) obtained from the same litter was used.

### Results

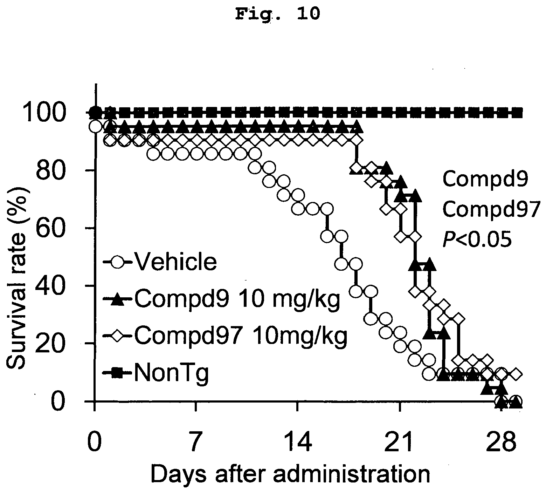

The results are shown in Fig. 10. As the Kaplan-Meier survival curve shows, all compounds showed a significant life prolonging effect on the life of CSQ TG mouse.
Compd 9: compound 9, Compd 97: compound 97
n = 21 (CSQ TG mouse), n = 5 (non-TG mouse)
compound 9, log-rank χ2 = 5.7672 *P < 0.05;
compound 97, log-rank χ2 = 5.9304 *P < 0.05.

### Formulation Example 1 (production of capsule, not according to the invention)

| | | |
|---|---|---|
| 1) | compound of Comparative Example 1 | 30 mg |
| 2) | fine powder cellulose | 10 mg |
| 3) | lactose | 19 mg |
| 4) | magnesium stearate | 1 mg |
| | Total | 60 mg |

| | | |
|---|---|---|
| 1), 2), 3) and 4) are mixed and filled in a gelatin capsule. | | |

### Formulation Example 2 (production of tablet, not according to the invention)

| | | |
|---|---|---|
| 1) | compound of Comparative Example 1 | 30 g |
| 2) | lactose | 50 g |
| 3) | cornstarch | 15 g |
| 4) | calcium carboxymethylcellulose | 44 g |
| 5) | magnesium stearate | 1 g |
| | 1000 tablets total | 140 g |

The total amount of 1), 2) and 3) and 4) (30 g) is kneaded with water, vacuum dried, and sieved. The sieved powder is mixed with 4) (14 g) and 5) (1 g), and the mixture is punched by a tableting machine, whereby 1000 tablets containing 30 mg of the compound of Example 1 per tablet are obtained.

### [Industrial Applicability]

The compound of the present invention has an IP6K inhibitory action and is expected to be useful as a prophylactic or therapeutic agent for diseases such as cardiac failure, diabetes and the like.

This application is based on patent application No. 2017-066579 filed in Japan (filing date: March 30, 2017.

## Claims

1. A compound which is 4-((3,5-Dichloropyridin-2-yl)oxy)-2'-oxo-1',2'-dihydrospiro[cyclohexane-1,3'-indole]-5'-carboxylic acid or a salt thereof.

2. A medicament comprising the compound according to claim 1 or a salt thereof.

3. The medicament according to claim 2, which is an inositol hexakisphosphate kinase inhibitor.

4. The medicament according to claim 2, which is a prophylactic or therapeutic agent for a disease selected from cardiac failure and diabetes.

5. The compound according to claim 1 or a salt thereof for use in the prophylaxis or treatment of a disease selected from cardiac failure and diabetes.

6. The compound according to claim 1 or a salt thereof for use in the prophylaxis or treatment of a disease by inhibiting inositol hexakisphosphate kinase.

## Patentansprüche

1. Verbindung, die 4-((3,5-Dichlorpyridin-2-yl)oxy)-2'-oxo-1',2'-dihydrospiro[cyclohexan-1,3'-indol]-5'-carboxylsäure oder ein Salz davon ist.

2. Medikament, umfassend die Verbindung nach Anspruch 1 oder ein Salz davon.

3. Medikament nach Anspruch 2, das ein Inositol-Hexakisphosphat-Kinase-Inhibitor ist.

4. Medikament nach Anspruch 2, das ein prophylaktisches oder therapeutisches Mittel für eine Krankheit ist, die aus Herzinsuffizienz und Diabetes ausgewählt ist.

5. Verbindung nach Anspruch 1 oder Salz davon zur Verwendung bei der Prophylaxe oder Behandlung einer Krankheit, die aus Herzinsuffizienz und Diabetes ausgewählt ist.

6. Verbindung nach Anspruch 1 oder Salz davon zur Verwendung bei der Prophylaxe oder Behandlung einer Krankheit durch Inhibieren der Inositol-Hexakisphosphat-Kinase.

## Revendications

1. Composé qui est l'acide 4-((3,5-dichloropyridin-2-yl)oxy)-2'-oxo-1',2'-dihydrospiro[cyclohexane-1,3'-indole]-5'-carboxylique ou un sel de celui-ci.

2. Médicament comprenant le composé selon la revendication 1 ou un sel de celui-ci.

3. Médicament selon la revendication 2, qui est un inhibiteur de la kinase d'inositol hexakisphosphate.

4. Médicament selon la revendication 2, qui est un agent prophylactique ou thérapeutique destiné à une maladie choisie parmi l'insuffisance cardiaque et le diabète.

5. Composé selon la revendication 1 ou sel de celui-ci, destiné à être utilisé dans la prophylaxie ou le traitement d'une maladie choisie parmi l'insuffisance cardiaque et le diabète.

6. Composé selon la revendication 1 ou sel de celui-ci destiné à être utilisé dans la prophylaxie ou le traitement d'une maladie par inhibition de la kinase d'inositol hexakisphosphate.
